# EUROPÄISCHE PATENTSCHRIFT

(11) **EP 2 211 730 B1**
(45) Veröffentlichungstag und Bekanntmachung des Hinweises auf die Patenterteilung: **05.06.2019**
(21) Anmeldenummer: 08839987.8
(22) Anmeldetag: 15.10.2008
(51) Int. Cl.: A61B 17/16, A61B 17/17

(54) **VORRICHTUNG ZUR KnochenBEARBEITUNG**
DEVICE for WORKING bone
DISPOSITIF DE TRAITEMENT D'OS

(30) Priorität: 17.10.2007 DE 102007050017
(43) Veröffentlichungstag der Anmeldung: 04.08.2010
(73) Patentinhaber: Orthotaxy, 38610 Gieres (FR)
(72) Erfinder: RADERMACHER, Klaus, 52222 Stolberg (DE); FOLLMANN, Axel, 52064 Aachen (DE); HEGER, Stefan, 52064 Aachen (DE)
(74) Vertreter: Regimbeau
(86) Internationale Anmeldenummer: PCT/EP2008/008699
(87) Internationale Veröffentlichungsnummer: WO 2009/049863

(56) Entgegenhaltungen:
- WO-A-2005/048852
- DE-A1- 19 914 455
- DE-A1-102004 035 001
- US-A- 6 033 409
- "VARIABLE DEPTH BLIND HOLE DRILLING", IBM TECHNICAL DISCLOSURE BULLETIN, INTERNATIONAL BUSINESS MACHINES CORP. (THORNWOOD), US, vol. 33, no. 3B, 1 August 1990 (1990-08-01), pages 150-154, XP000124304, ISSN: 0018-8689

## Beschreibung

Die Erfindung betrifft eine Vorrichtung zur Materialbearbeitung, umfassend einen Werkzeugkopf und ein daran angeordnetes Werkzeug. Die Erfindung betrifft weiterhin ein Verfahren zur Materialbearbeitung, bei dem ein Werkzeugkopf mit einem daran angeordneten Werkzeug entlang der Oberfläche eines zu bearbeitenden Materials geführt wird.

Die beschriebene Vorrichtung und das Verfahren sollen dabei geeignet sein, allgemein jegliche Art von Material zu bearbeiten, insbesondere hier auch Gewebe- oder Knochenmaterialien und somit auch Anwendung finden können in der Medizintechnik.

Bei der Materialbearbeitung, insbesondere in der Medizin und hier insbesondere bei der Bearbeitung von Gewebe und Knochen ist es wesentlich, Informationen über die lokalen Eigenschaften des zu bearbeitenden Materials vorliegen zu haben. Beispielsweise wird bei der sogenannten Trepanation der Schädel eröffnet, z.B. um neurochirurgische Operationen durchführen zu können, wobei ein knöcherner Zugang in das Schädelinnere eines Lebewesens, wie beispielsweise einer menschlichen Person, geschaffen wird.

Dabei ist es vorrangiges Ziel, die am Schädelknochen sehr eng anliegende harte Hirnhaut, die Dura Mater, zu schützen, d.h. jegliche Verletzung durch das eingesetzte Werkzeug zu vermeiden, da Verletzungen der Hirnhaut zu einer Verlängerung der Operationszeit sowie auch der postoperativen Wundheilung sowie zu einem erhöhten Infektionsrisiko führen können. Darüber hinaus sind bei der Verletzung der Hirnhaut weiterreichende Komplikationen (Wundheilung, Mortalität,...) und erhöhte Behandlungskosten zu berücksichtigen.

Im Stand der Technik ist es weiterhin bekannt, zur Schaffung eines Zugangs in das Schädelinnere spezielle Bohrer einzusetzen, mit denen mehrere Bohrungen entlang einer Resektionslinie erzeugt werden. Diese Bohrungen können anschließend mit einem weiteren Instrument bzw. Werkzeug, dem sogenannten Kraniotom, verbunden werden, um so ein herausnehmbares Knochenareal zu erzeugen. Bei einem solchen Kraniotom handelt es sich üblicherweise um ein Fräsinstrument mit z.B. leicht konischem Fräser, mit welchem der Schnitt herbeigeführt wird.

Hierbei ist es bekannt, den Fräser an seinem abtriebseitigen Ende mit einem abgewinkelten Schuh zu lagern und ihn dadurch zumindest theoretisch von der Hirnhaut zu trennen. Dieser Schuh dient als Schutz für die Hirnhaut und soll im zu schneidenden Areal dafür sorgen, dass die Hirnhaut kontinuierlich vom Knochen abgeschoben und somit aus dem Schnittbereich verdrängt wird.

Hierbei ist es jedoch bekannt, dass dieser Wirkmechanismus versagen kann, z.B. regelmäßig bei lokalen Dickeschwankungen des Schädelknochens sowie einer Adhäsion der Hirnhaut am Schädelknochen. Es kann sodann zu einem Reißen der Hirnhaut in einem nicht sichtbaren Bereich kommen, wodurch eine unmittelbare Reaktion auf eine solche Schädigung der Hirnhaut nicht möglich ist und die Schädigung somit oftmals bis zum Abschluss des Schneidvorgangs fortgeführt wird. Während einer Operation ist eine solche Verletzung gezielt zu beheben, wofür oftmals eine notwendige Naht oder Duraplastik neben einer signifikanten Verlängerung der Operationszeit auch die Gefahr einer Liquorfistel und damit das Risiko einer postoperativen Infektion mit deutlicher Erhöhung der Sterblichkeit sowie Erhöhung der Kosten nach sich ziehen kann.

Nach Entnahme eines Knochendeckels können weitere operative Maßnahmen durchgeführt werden und im Anschluss an diese Operation kann der Knochendeckel in der Regel wieder eingesetzt werden, sofern er nicht durch Tumorwachstum, Trauma oder ähnliches zerstört ist. Die eingangs beschriebene Schnitttechnik führt dabei zu einer verbleibenden knöchernen Lücke, die auf einen Schnittdefekt zurückzuführen ist aufgrund der Bohrlöcher sowie dem Sägespalt. In anderen medizinischen Bereichen, wie z.B. der Mund-, Kiefer- oder Gesichtschirurgie sowie auch der Wirbelsäulenchirurgie ergeben sich vergleichbare Problemstellungen des Weichgewebeschutzes.

Die nachfolgend beschriebene Vorrichtung sowie auch das Verfahren ist jedoch nicht beschränkt auf den Einsatz der Materialbearbeitung in medizinischen Anwendungsgebieten. Ein nicht erfindungsgemäßes Beispiel der Vorrichtung und eines Verfahrens der nachfolgend beschriebenen Art können allgemein eingesetzt werden, um jegliche Art von Material bearbeiten zu können, also z.B. auch bei der Bearbeitung von nicht lebenden Körpern, insbesondere von Hohlformen oder flächigen Gebilden.

Die deutsche Patentanmeldung DE 101 17 403 beschreibt weiterhin auch ein Verfahren und eine Vorrichtung, um einen Materialabtrag oder eine Materialbearbeitung zu kontrollieren. Hier wird es beschrieben, ein Werkzeug zur manuellen Führung in Abhängigkeit von der Geometrie eines vorgegebenen Objektvolumens und einer vorgegebenen zu erzielenden Objektform hinsichtlich der Materialabtragsleistung zu regeln oder zu steuern, d.h. hier insbesondere die Leistung eines Fräs- oder Bohrwerkzeugs in Abhängigkeit von Navigationskoordinaten zu ändern und beispielsweise dann, wenn die erzielte Objektform erreicht ist, das Werkzeug abzuschalten.

Bei einer derartigen Art der Materialbearbeitung wird es jedoch als nachteilig empfunden, dass die Leistung des verwendeten Werkzeugs aufgrund dieses bekannten Verfahrens bzw. der bekannten Vorrichtung variiert, wobei es gerade bei Schneid- oder Fräswerkzeugen bekannt ist, dass eine Leistungsverringerung, gegebenenfalls sogar bis zum Stillstand innerhalb eines zu bearbeitenden Materials zu einem Festsetzen des Werkzeugs innerhalb des Materials führen kann. Gerade in medizinischen Anwendungen sowie auch allgemein in jeglicher Art der Anwendung bei der Materialbearbeitung stellt dies einen erheblichen Nachteil dar, da in dem Augenblick des Festsetzens ein solches Werkzeug außer Kontrolle geraten kann. Allenfalls kann ein solches vorbekanntes Gerät zur Materialbearbeitung nur mit einer ausreichenden Sicherheit bei einer solchen Art von Bearbeitung eingesetzt werden, der ein rotierendes Bearbeitungsprinzip zugrunde liegt, wie beispielsweise Bohren oder Fräsen, nicht jedoch bei einer Bearbeitung wie dem Sägen.

Das Dokument WO 2005/048852 beschreibt ein Gerät zur Bahnregelung eines medizinischen Instruments.

Das Dokument DE 199 14455 beschreibt ein Verfahren zur Bestimmung der Bewegung eines Körperorgans oder Therapiegebiets eines Patienten.

Das Dokument DE 10 2004 035001 beschreibt eine Knochenschneidevorrichtung mit einem Impedanzmessinstrument zum Messen der elektrischen Impedanz um den Werkzeugkopf, so dass die verbleibende Dicke des verbleibenden Materials gemessen wird.

Das Dokument US 6,033,409 offenbart eine Vorrichtung gemäß dem Oberbegriff des Anspruchs 1.

Das Dokument "Variable depth blind hole drilling", IBM Technical Disclosure Bulletin, International Business Machines Corp. (Thornwood) US, Bd. 33, Nr. 3B, 01.08.1990, Seiten 150-154, XP000124304, ISSN: 0018-8689 beschreibt eine Vorrichtung zum Bohren von Sacklöchern in gedruckte Leiterplatten.

Aufgabe der Erfindung ist es, für jegliche Art der Knochenbearbeitung eine Vorrichtung bereitzustellen, bei dem während der Durchführung der Knochenbearbeitung eine automatisierte Kontrolle der Lage des Werkzeugs durchgeführt werden kann. Dies soll bei einer jeglichen Art der Knochenbearbeitung insbesondere im medizinischen Anwendungsfeld, für ein präzises und insbesondere sicheres Bearbeitungsergebnis Sorge tragen.

Gelöst wird diese Aufgabe mit einer Vorrichtung gemäß Anspruch 1.

Wesentlicher Kerngedanke der Erfindung ist es somit, eine Vorrichtung zur Verfügung zu stellen, bei dem ein Werkzeugkopf zum Einsatz kommt, an dem ein Werkzeug angeordnet ist, wobei die Winkellage des Werkzeugs durch mindestens zwei Stützelemente am Werkzeugkopf beeinflussbar ist.

Der Werkzeugkopf kann dabei einen Antrieb aufweisen, um das Werkzeug anzutreiben. Bei einem solchen Antrieb kann es sich z.B. um einen solchen handeln, der ein Werkzeug in Bewegung setzt oder lediglich ein Werkzeug mit der benötigten Energie versorgt. So wird hierbei unter einem Werkzeug jegliches Element oder auch Vorrichtung verstanden, welches geeignet ist, ein Material zu bearbeiten.

Hierbei kann es sich grundsätzlich um jegliche Art der Bearbeitung handeln, wie beispielsweise Trennen oder auch Fügen. Z.B. können als Werkzeuge Sägen, Fräsen, Bohrer, Schleifoder Hobelelemente zum Einsatz kommen, ebenso wie Hochfrequenzskalpelle, Schneiden und Messer sowie auch Werkzeuge bzw. Vorrichtungen, die Laserstrahlen oder Wasserstrahlen einsetzen und solche Werkzeuge, die zum Kleben, Schweißen oder Löten Einsatz finden.

Die Liste der möglichen Werkzeuge ist hier grundsätzlich beliebig offen und variiert je nach Art der gewünschten Knochenbearbeitung wobei der jeweilige Werkzeugkopf angepasst ist, um das daran angeordnete Werkzeug in Betrieb zu nehmen, wofür im oder am Werkzeugkopf beispielsweise der eingangs genannte Antrieb oder sonstige Einrichtungen vorgesehen sein können, um ein Werkzeug in Betrieb zu nehmen, sei es auch nur, um dieses mit der notwendigen Energie zu versorgen.

Weiterhin ist es ein wesentlicher Aspekt der Erfindung, dass ein Werkzeugkopf mit dem daran angeordneten Werkzeug nicht lediglich alleine durch äußere Maßnahmen geführt ist, wie beispielsweise rein manuell durch eine Bedienperson oder aber auch durch eine übergeordnete Robotersteuerung, sondern dass es hier gemäß der Erfindung vorgesehen ist, wenigstens zwei verstellbaren Stützelemente am Werkzeugkopf vorzusehen, welche relativ zum Werkzeugkopf verstellt werden können und über welche der Werkzeugkopf während der Bearbeitung des Knochens an der Knochenoberfläche abgestützt ist bzw. wird.

So kann durch die Stützelemente der Abstand des Werkzeugkopfes zur Oberfläche des zu bearbeitenden Knochens beeinflusst werden, wofür eine Verstellung des Stützelementes relativ zum Werkzeugkopf erfolgt. Für die Verstellung der Stützelemente ist es dabei vorgesehen, dass im oder am Werkzeugkopf für jedes der vorgesehenen Stützelemente oder auch für gegebenenfalls mehrere gemeinsam wenigstens ein Aktor vorgesehen ist.

Ein solcher Aktor wird angesteuert durch eine Steuer- oder Regeleinheit, welcher die notwendigen Daten zur Verstellung des Stützelementes während dieses Vorgangs der Knochenbearbeitung zugeführt werden. Eine solche Einheit kann von dem Werkzeugkopf umfasst sein oder auch extern angeordnet sein.

Solche genannten Stützelemente, welche an einem Werkzeugkopf zusätzlich zu einem Werkzeug angeordnet sind, können in einer möglichen und bevorzugten Ausführungsform z.B. als ein Stempel ausgebildet sein, der mittels eines Aktors aus dem Werkzeugkopf aus- oder in diesen einfahrbar ist. So kann bereits in einer nicht erfindungsgemäßen Ausführungsform bei Vorhandensein von nur einem solchen Stützelement die Lage des Werkzeugs, hier insbesondere die Interaktionstiefe des Werkzeugs mit dem zu bearbeitenden Material, während des Vorgangs der Materialbearbeitung eingestellt werden, wofür wie eingangs genannt dem steuernden oder regelnden Aktor die notwendigen Daten zugeführt werden.

Erfindungsgemäß sind dabei mehr als nur ein Stützelement, beispielsweise zwei oder drei oder noch mehr Stützelemente, vorgesehen, die insbesondere so angeordnet sein können, dass sie ein Werkzeug, welches zur Bearbeitung des Materials vorgesehen ist, umgeben. So kann neben der Interaktionstiefe des Werkzeugs mit dem zu bearbeitenden Material die Lage auch noch in anderen Freiheitsgraden, wie beispielsweise die Neigungswinkel in mehreren Dimensionen relativ zur Materialoberfläche eingestellt werden, dadurch dass die Stützelemente unterschiedlich verstellt werden und sich so bei gleichzeitiger Anlage des Werkzeugkopfes über diese Stützelemente an der Materialoberfläche der Winkel des Werkzeugkopfes zu dieser und somit auch der Winkel des Werkzeuges ändert. Gerade die Winkelverstellung ist gegenüber der Höhenverstellung bei Werkzeugen wie Laserstrahlen oder Wasserstrahlen wichtig.

In einer anderen nicht erfindungsgemäßen Ausführung kann ein Stützelement auch als eine Hülse ausgebildet sein, die ein Werkzeug umgibt. Auch bei dieser Ausführung kann das hülsenförmige Stützelement mittels eines Aktors aus dem Werkzeugkopf aus- oder in diesen einfahrbar sein. Insbesondere bei rotierenden Werkzeugen, wie Bohrern oder Fräsern, kann eine solche Ausführung eines Stützelementes bevorzugt sein, wohingegen eine Ausführung mit den beispielhaft eingangs genannten Stempeln und somit lokal vom Werkzeug getrennten Stützelementen bei Werkzeugen bevorzugt vorgesehen sein kann, die eine von der Rotation abweichende Arbeitsbewegung ausführen, wie z.B. eine Oszillationsbewegung, wie sie für Sägewerkzeuge eingesetzt werden kann.

Gemäß einer möglichen Ausgestaltung eines Verfahrens sowie auch der erfindungsgemäßen Vorrichtung kann es vorgesehen sein, dass die Daten, die dem wenigstens einen Aktor des wenigstens einen Stützelementes während der Bearbeitung zugeführt werden, in Abhängigkeit einer festgestellten Position des Werkzeugkopfes relativ zu dem zu bearbeitenden Material zugeführt werden.

Zur Feststellung einer Position kann es dabei vorgesehen sein, dass die erfindungsgemäße Vorrichtung mit einem Navigationssystem zusammenwirkt, mittels dem die Position des Werkzeugkopfes und somit auch gleichzeitig die Position des daran angeordneten Werkzeugs relativ zu einem zu bearbeitenden Material ermittelbar ist. Ist während der Bearbeitung jeweils die Position des Werkzeugkopfes durch eine solche Feststellung bekannt, so können entsprechende positionsabhängige Daten, z.B. aus einer Datenbank, dem wenigstens einen Aktor des wenigstens einen Stützelementes zugeführt werden, um so die Lage des Werkzeugs während der Bearbeitung einzustellen.

Zur Feststellung der Position kann es dabei vorgesehen sein, dass am Werkzeugkopf und/oder am Material mehrere, durch eine Kamera oder ein anderes räumliches Lokalisierungssystem erfassbare Markierungen angeordnet sind. Hierbei kann im Wesentlichen auf übliche Navigationstechniken zurückgegriffen werden.

Z.B. aus den in einem Bild erfassten Markierungen sowohl des Werkzeugkopfes als auch des Materials kann so auf die relative Position dieser beiden Elemente zueinander geschlossen werden, wobei die aus der Datenbank stammenden Daten in einer Ausführung, z.B. vor einer Materialbearbeitung durch messtechnische, insbesondere bildgebende Erfassung des zu bearbeitenden Materials positionsabhängig erfasst und gespeichert sein können.

Beispielsweise kann es hierfür vorgesehen sein, die Daten radiologisch zu erfassen, so dass bestimmte Eigenschaften des zu bearbeitenden Knochens positionsabhängig in der Datenbank vorliegen können.

Bei den möglichen Eigenschaften, die während einer Knochenbearbeitung berücksichtigt werden sollen und in deren Abhängigkeit die Daten durch diese vorgenannte Ausführungsform oder auch die nachfolgend genannten Ausführungsformen erfasst werden, kann es sich z.B. um die Dicke des Materials am Ort oder in der Umgebung des Ortes der Knochenbearbeitung handeln, ebenso wie andere Eigenschaften, wie beispielsweise grundsätzlich jegliche mechanische Eigenschaften, wie Abmessungen, Dichte oder auch elektrische Eigenschaften, wie Widerstände, Impedanzen oder sonstige jegliche Art von Eigenschaft, die für die Art der Materialbearbeitung von Interesse sein kann.

Es ist auch vorgesehen, dass die Vorrichtung wenigstens eine Sensorik aufweist, mittels der bei der Materialbearbeitung Daten zur Ansteuerung des wenigstens eines Aktors der Stützelemente erfassbar sind. So wird es hier als wesentlich empfunden, dass in situ, d.h. während der Durchführung der Knochenbearbeitung mittels wenigstens einer solchen Sensorik, insbesondere einer solchen, die am/im Werkzeugkopf oder am/im Werkzeug selbst angeordnet ist, die nötigen Daten erfasst werden, wobei diese Daten dann Informationen über Eigenschaften am Ort oder zumindest in der Umgebung des Ortes der Materialbearbeitung repräsentieren, wie die z.B. eingangs genannte Dicke oder auch andere Eigenschaften.

So besteht hier die Möglichkeit, grundsätzlich eine Knochenbearbeitung unabhängig von einer vorherigen Erfassung der notwendigen Eigenschaften des Materials durchzuführen oder aber in einer weiterhin möglichen Ausführung, bei der beide vorgenannten Varianten kombiniert werden, die Möglichkeit, dass die Daten in Abhängigkeit einer festgestellten Position des Werkzeugkopfes, die beispielsweise aus der eingangs genannten Datenbank stammen, korrigiert werden mit denjenigen Daten, die bei der Materialbearbeitung mittels einer Sensorik am Werkzeugkopf erfasst werden. Beispielsweise erschließt sich so die Möglichkeit, die Steuerung einer automatischen Wegführung des Werkzeugkopfes anhand der unmittelbar am oder in der Umgebung des Ortes des Werkzeugs erfassten Daten zu korrigieren. Hierdurch kann eine erhöhte Sicherheit bei einer automatischen Wegführung realisiert werden.

Die eingangs genannte Sensorik kann dabei wenigstens einen Sensor umfassen, um eine Eigenschaft des zu bearbeitenden Materials messtechnisch erfassbar zu machen. Hierbei kann es auch vorgesehen sein, dass die Sensorik ein aktives Element umfasst, durch dessen Aktion erst die Erfassung der bestimmten Eigenschaft messtechnisch möglich wird.

So kann eine solche Sensorik beispielsweise auf einem akustischen Messprinzip basieren und zur Durchführung einer Messung einer gewünschten Eigenschaft, beispielsweise einen Ultraschallsensor und/oder einen Ultraschallempfänger aufweisen. Es besteht so in einer Ausgestaltung der Erfindung die Möglichkeit, mittels einer solchen genannten Sensorik durch Ultraschall sensierbare Eigenschaften, wie beispielsweise die Dicke eines zu bearbeitenden Materials am Ort oder zumindest in der Umgebung des Ortes der Materialbearbeitung zu erfassen und anhand einer so erfassten Eigenschaft Daten zu bilden, die der Steuer- bzw. Regeleinheit des Aktors bzw. den mehreren Aktoren zugeführt werden, um die Lage des Werkzeugs zu bestimmen. Ein solche, zuvor beschriebene Sensorik sowie auch jegliche andere mögliche Art von Sensorik zur Erfassung grundsätzlich beliebiger Knocheneigenschaften kann grundsätzlich irgendwo am Werkzeugkopf und somit der erfindungsgemäßen Vorrichtung angeordnet sein. Wesentlich ist, dass mittels einer solchen Sensorik die Möglichkeit besteht, die gewünschte Eigenschaft am Ort oder zumindest in der Umgebung des Ortes der Knochenbearbeitung zu erfassen. Hierfür kann es in einer bevorzugten Ausführung vorgesehen sein, dass die Sensorik zumindest teilweise in wenigstens einem der Stützelemente angeordnet ist, beispielsweise im Bereich des Ende eines Stützelementes, welches der Oberfläche eines zu bearbeitenden Materials zugewandet ist. So kann insbesondere, wenn erfindungsgemäß ein Werkzeugkopf mittels wenigstens zweier solcher Stützelemente an der Oberfläche des zu bearbeitenden Knochens aufgestützt wird, über wenigstens eine Kontaktstelle eine Messung zur Bestimmung der Eigenschaft, wie beispielsweise der Dicke, vorgenommen werden.

Dabei kann eine vollständige Sensorik oder aber auch lediglich eine Sensorik teilweise in einem solchen Stützelement angeordnet sein, so dass die Erfassung der Eigenschaft durch dieses wenigstens eine Stützelement alleinig oder aber auch durch die Zusammenwirkung von zwei Sensorikteilen, die auf unterschiedliche Stützelemente aufgeteilt sein können, erfolgen kann.

Beispielsweise kann es unter Rückbezug auf eine nach dem Ultraschallprinzip arbeitende Sensorik vorgesehen sein, dass in einem der Stützelemente ein Ultraschallsender und in einem anderen Stützelement ein Ultraschallempfänger angeordnet ist. Selbstverständlich besteht die Möglichkeit, innerhalb eines Stützelementes immer eine vollständige Sensorik, d.h. in diesem Anwendungsfall beispielsweise Ultraschallsender und Ultraschallempfänger gemeinsam anzuordnen, wobei es verfahrenstechnisch vorgesehen sein kann, dass die von der Sensorik wenigstens eines der Stützelemente ausgesendeten Ultraschallimpulse von derselben Sensorik oder aber von der Sensorik eines anderen Stützelementes als Echos empfangbar sind.

Bei der genannten Ausbildung einer Sensorik, die auf dem Ultraschallmessprinzip beruht, kann es ergänzend auch vorgesehen sein, dass eine Sensorik, insbesondere ein Stützelement mit einer solchen Sensorik, einen Applikator zur Applikation eines Koppelfluids aufweist, um eine bessere Einkopplung von Ultraschallimpulsen in die Materialoberfläche des zu bearbeitenden Materials zu gewährleisten.

Neben der hier genannten Möglichkeit, eine Sensorik nach dem Ultraschallprinzip zu verwenden, können selbstverständlich auch andere Arten von Sensorik eingesetzt werden, die andere Messprinzipien verwenden, um die vorgenannte Eigenschaft, wie z.B. die Dicke oder auch andere Eigenschaften, wie elektrische Widerstände oder Impedanzen, am Ort oder in der Umgebung des Ortes der Materialbearbeitung zu erfassen.

Hier kann es auch vorgesehen sein, dass bei mehreren Stützelementen, die um ein Werkzeug herum angeordnet sind, Daten von mehreren Sensoren erfasst werden, die jeweils Teil einer solchen Sensorik sind, die sodann ebenfalls um ein Werkzeug herum angeordnet sind, wobei es vorgesehen sein kann, die Messdaten der mehreren Sensoren zu verarbeiten, beispielsweise durch eine Datenverarbeitungsanlage, um so auf eine Eigenschaft am Ort des Werkzeugs zu schließen. So kann also beispielsweise die gewünschte Eigenschaft an mehreren Stellen um ein Werkzeug herum vermessen werden, wobei sich das Werkzeug beispielsweise in einem geometrischen Bezugspunkt, z.B. Mittelpunkt, bezogen auf die Anordnung von mehreren Sensoren, die in Stützelementen angeordnet sein können, angeordnet ist.

In einer anderen möglichen Ausgestaltung der Sensorik kann es auch vorgesehen sein, dass diese direkt in dem Werkzeug selbst angeordnet ist, so dass die Vermessung der gewünschten Eigenschaft unmittelbar am Ort des Werkzeugs mit einer hohen Genauigkeit stattfinden kann.

In einer Ausführung kann es auch vorgesehen sein, dass der Werkzeugkopf auch eine Sensorik umfasst, mittels der Daten betreffend die Navigation erfasst werden können. Eine solche Sensorik kann ein vorgenanntes Navigationssystem ersetzen oder auch ergänzen. Beispielsweise können durch die Navigationsdaten, die mit der Sensorik im / am Werkzeugkopf erfasst werden die Daten eines zum Werkzeugkopf externen Navigationssystems, wie beispielsweise der eingangs genannten Art ergänzt und/oder korrigiert werden. Eine solche Sensorik kann z.B. wenigstens einen Inertialsensor umfassen, mittels dem Beschleunigungswerte aufnehmbar sind. So kann z.B. die Neigung und/oder auch die Position des Werkzeugkopfes erfasst werden. Eine solche Sensorik kann auch optischer Art sein, mittels der Relativbewegungen zum bearbeitenden Material erfasst werden können.

Bei einer weiterhin möglichen Ausgestaltung der Vorrichtung kann es auch vorgesehen sein, dass weitere Daten, die dem Aktor zugeführt werden, um diese Stützelemente relativ zum Werkzeugkopf zu verstellen, von einer Einheit stammen, die extern zum Werkzeugkopf angeordnet ist und mit der ein persönliches Empfinden einer Person registriert werden kann, an welcher die erfindungsgemäße Vorrichtung zur Durchführung des Verfahrens angewendet wird.

So besteht beispielsweise die Möglichkeit, einer behandelten Person eine Einheit zur manuellen Handhabung zu überreichen, mittels der die Person sein Schmerzempfinden oder ein Unbehaglichkeitsempfinden, beispielsweise durch das Drücken eines Knopfes, kundtun kann. Ein so erhaltenes Signal, z.B. in Abhängigkeit des Knopfdruckes kann an die erfindungsgemäße Vorrichtung und hier insbesondere die Steuer- bzw. Regeleinheit des wenigstens einen Aktors weitergeleitet werden, um zu bewirken, dass die Interaktionstiefe des Werkzeugs z.B. durch Ausfahren des wenigstens einen Stützelementes verringert wird.

Eine mögliche nicht erfindungsgemäße Anwendung ist beispielsweise das Zerschneiden von Gips, wobei sichergestellt sein muss, dass durch das Zersägen eines Gipsverbandes mit einer bestimmten Dicke die darunter liegende Haut eines Patienten geschützt wird. In dem Augenblick, wo ein Patient verspürt, dass ein Werkzeug zum Auftrennen des Gipses, insbesondere ein Oszillationswerkzeug, wie beispielsweise eine Oszillationssäge, in Kontakt oder nahe der Haut der Person kommt, kann durch die zugeführten Daten aufgrund der Aktion des Patienten bewirkt werden, dass ein Stützelement am Kopf ausgefahren und hierdurch die Interaktionstiefe des Werkzeugs mit dem Gips verringert wird.

Eine derartige, auf Personenwirkung vorgesehene Rückkopplung und Zurverfügungstellung von Daten zwecks Ansteuerung des wenigstens einen Aktors kann nicht nur bei diesem Beispiel, sondern bei grundsätzlich jeglicher Art von Maßnahmen zum Einsatz kommen, bei welchen eine Person bei Bewusstsein ist und somit die Rückkopplung vornehmen kann.

Bei jeder der eingangs genannten verschiedenen Möglichkeiten, die Vorrichtung auszugestalten, kann grundsätzlich jegliche Art der Knochenbearbeitung erfolgen. Eine besonders bevorzugte Anwendung des der erfindungsgemäßen Vorrichtung kann erfolgen bei der Materialbearbeitung zur Öffnung des Schädelknochens eines Lebewesens, da hier erfindungsgemäß die Möglichkeit besteht, durch eine vorgelagerte radiologische Erfassung eines Patientenschädels ortsabhängig die Schädelknochendicke zu erfassen und kumulativ die Dicke des Schädelknochens durch eingangs genannte Sensorik messtechnisch während der Durchführung der Schädeleröffnung der erfindungsgemäßen Vorrichtung zur Verfügung zu stellen, um die Stützelemente zu steuern bzw. zu regeln.

Andere nicht erfindungsgemäße Anwendungsgebiete betreffen beispielsweise auch den Materialabtrag zur Erzielung gleichmäßiger Dicken des Materials entlang eines Bearbeitungsweges bei grundsätzlich beliebigen Arten von Materialien, wie beispielsweise auch Metallen, Kunststoffen oder sonstigem.

Ausführungsbeispiele mit Bezug auf eine medizinische Anwendung sind in den nachfolgenden Figuren dargestellt. Es zeigen:
- Figur 1: eine erfindungsgemäße Vorrichtung zur Trepanation des Schädelknochens
- Figur 2: nicht erfindungsgemäße Vorrichtungen zur Durchführung von Bohrungen oder Fräsungen gewünschter Tiefe
- Figur 3: Ein Datenverarbeitungsschema.

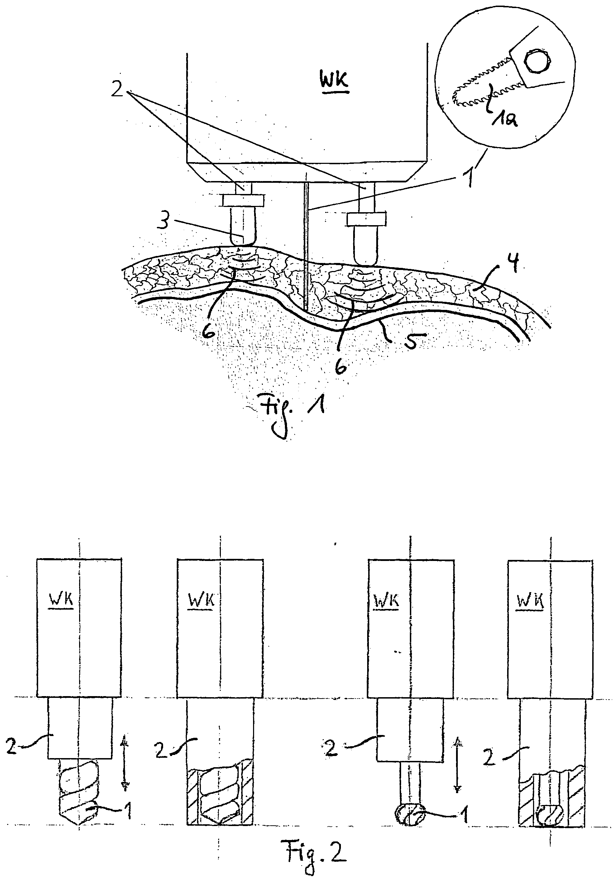

Die Figur 1 zeigt in einer seitlichen Schnittdarstellung einen erfindungsgemäßen Werkzeugkopf WK, an dem ein Werkzeug 1 angeordnet ist, welches in diesem Fall gemäß der erläuternden weiteren Darstellung als Sägeblatt 1a ausgebildet ist, um somit in einen Schädelknochen 4, der von der außenliegenden Haut befreit wurde, Sägeschnitte einzubringen mit einer Schnittbreite, die der Sägeblattdicke entspricht.

Um hier zu verhindern, dass während des Schneidvorgangs zum Auftrennen des Schädelknochens eine Verletzung der unter dem Schädelknochen 4 angeordneten Hirnhaut 5 stattfindet, ist es hier erfindungsgemäß vorgesehen, am Werkzeugkopf beispielsweise zwei Stützelemente 2 anzuordnen, die beidseitig der Sägeblattfläche des Sägeblattes 1 angeordnet sind und somit das Werkzeug 1 umgeben. Die Stützelemente 2 sind dabei durch hier nicht dargestellte Aktoren innerhalb des Werkzeugkopfes WK derart verstellbar, dass diese aus dem Werkzeugkopf mittels dieser Aktoren herausgefahren oder in den Werkzeugkopf hineingefahren werden können, so dass der Abstand des jeweils unteren Endes jedes der Stützelemente 2 variabel eingestellt werden kann.

Dies kann hier gemäß einer Ausführung derart erfolgen, dass die Position des Werkzeugs 1 durch ein externes Navigationssystem erfasst wird und Dicken betreffend die Schädeldicke des Schädelknochens 4 am Ort des Werkzeugs aus einer Datenbank ausgelesen werden, um so die notwendigen Informationen zur Ansteuerung der Stützelemente 2 den Aktoren des Werkzeugkopfes zuzuführen.

In der hier dargestellten Ausführung, die mit dieser vorgenannten auch kombinierbar ist oder alternativ eingesetzt werden kann, ist es vorgesehen, dass die Stützelemente, hier z.B. an ihrem jeweiligen unteren Ende eine Sensorik 3 aufweisen, die Ultraschallsender und Ultraschallempfänger umfassen. Die jeweilige Sensorik 3 am unteren Ende der Stützelemente 2, mit denen die Stützelemente die Oberfläche des Schädelknochens 4 kontaktieren, sendet hier eine Ultraschallwelle 6, z.B. pulsartig aus, die am inneren Ende des Schädelknochens nach Durchlaufen der Dickes des Schädelknochens reflektiert und als Echo vom Ultraschallempfänger der Sensorik 3 empfangen werden kann. In Kenntnis der Schallgeschwindigkeit innerhalb des Schädelknochens kann so auf die Dicke des Schädelknochens jeweils am Ort der Einstrahlung der Ultraschallimpulse in den Schädelknochen oder auch durch rechentechnische Verarbeitung in Gebieten zwischen den Einstrahlungsorten und bevorzugt am Ort des Werkzeugs 1 geschlossen werden. Die so ermittelten Dickeninformationen können, sofern nötig, gewandelt werden in Ansteuerungsinformationen zur Steuerung der Aktoren der Stützelemente 2, um so die Einschnitttiefe des Werkzeugs 1 in den Schädelknochen 4 zu steuern bzw. auch zu regeln. Es kann so sicher gestellt werden, dass zwischen dem unteren Ende des Werkzeugs 1 und der Hirnhaut 5 optional ein Sicherheitsabstand verbleibt, um eine Verletzung der Hirnhaut 5 zu vermeiden, wobei jedoch ein vollständiges Durchtrennen des Schädelknochens 4 gewährleistet wird.

Ändert sich bei Sägefortschritt entlang einer gewünschten Trajektorie die Dicke des Schädelknochens am Ort des Werkzeugs, so wird dies in situ durch die Sensorik 3 in den Stützelementen 2 festgestellt, so dass durch entsprechende Ansteuerung der Aktoren der Stützelemente 2 ein weiteres oder geringeres Eintauchen des Sägeblattes in den Schädelknochen bewirkt werden kann, je nachdem welche Dicke der Schädelknochen am Ort des Werkzeugs bzw. in dessen Umgebung festgestellt wird.

Die Figur 2 zeigt ein Bohrwerkzeug 1 oder ein Fräswerkzeug 1. Hierbei ist das Stützelement 2 jeweils als eine Hülse ausgebildet, die das Werkzeug 1 vollständig umgibt. Je nach der Weite des Ausfahrens der Hülse 2 aus dem Werkzeugkopf WK ergibt sich durch den Abstand zwischen der Bohrspitze bzw. Fräskopfspitze und dem unteren Ende der Hülse 2 die Eintauchtiefe des Bohr- oder Fräswerkzeugs 1 in ein zu bearbeitendes Material.

Hierbei kann durch das Ausfahren der Hülse 2 die Eintauchtiefe auch vollständig minimiert werden, wie es in den jeweils rechtsseitigen Darstellungen zum Bohrwerkzeug oder Fräswerkzeug gezeigt ist. Praktisch wird dabei erreicht, dass dann, wenn das untere Ende der Hülse 2 auf der zu bearbeitenden Materialoberfläche aufliegt, das Bohr- oder Fräswerkzeug 1 aus dem Material zurückgezogen wird, dadurch, dass die Hülse aus dem Werkzeugkopf ausfährt. Die entsprechenden Daten können auch hier durch eine übergeordnete Steuerung, z.B. anhand von Navigationsdaten und festgestellten Positionen relativ zur Materialoberfläche zugeführt werden. Ebenfalls besteht auch hier grundsätzlich die Möglichkeit, dass eine Sensorik im Werkzeugkopf oder in der Hülse angeordnet ist, wobei beispielsweise auch die Hülse als Wellenleiter für einen Ultraschallimpuls eingesetzt werden kann.

Der Einsatz als Wellenleiter gilt grundsätzlich auch ebenso bei einer Ausführung gemäß der Figur 1 bzw. allgemein bei jeglicher Ausbildung von Stützelementen, so dass eine Sensorik nicht zwingend innerhalb eines Stützelementes angeordnet sein muss, sondern die Anordnung innerhalb des Werkzeugkopfes ausreichend ist, wobei dann die erzeugten Ultraschallimpulse oder auch sonstige Messsignale zur Bestimmung einer Eigenschaft des Materials durch die Stützelemente als Signalleiter ausgesendet und ebenso, insbesondere bei den akustischen Messprinzipien auch Echos empfangen werden können.

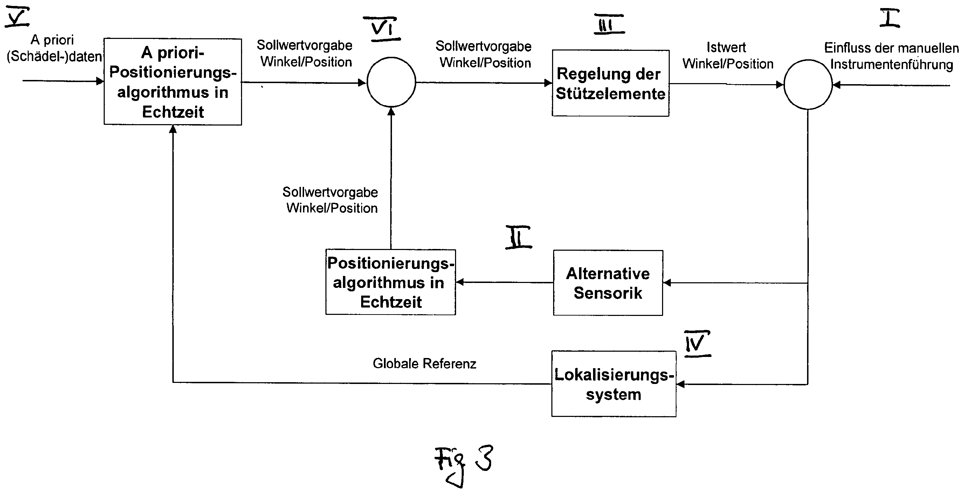

Die Figur 3 zeigt schematisch ein mögliches Prinzip zur Durchführung eines Verfahrens am Beispiel einer manuellen Führung der erfindungsgemäßen Vorrichtung.

In einem Schritt I können die bisherigen Werte der Positionierung der Vorrichtung, z.B. bzgl. kartesischen Koordinaten und/oder Winkel durch die manuelle Führung der Vorrichtung geändert werden. In einem Schritt II können durch Messung mittels der Sensorik neue Positionierungsvorgaben erstellt und im Schritt III eingestellt oder eingeregelt werden.

Hierbei besteht auch die Möglichkeit eines Abgleichs mit einem Lokalisierungssystem, welches im Schritt IV die Position der Vorrichtung bestimmen kann. Anhand der Bestimmten Positionen können aus einer Datenbank im Schritt V benötigte Daten hinzugeladen werden und mit den Daten der Sensorik verknüpft werden im Schritt VI. Die Verknüpften Daten dienen dann zur Steuerung oder Regelung der Stützelemente.

Bezüglich sämtlicher Ausführungen ist festzustellen, dass die in Verbindung mit einer Ausführung genannten technischen Merkmale nicht nur bei der spezifischen Ausführung eingesetzt werden können, sondern auch bei den jeweils anderen Ausführungen. Sämtliche offenbarten technischen Merkmale dieser Erfindungsbeschreibung sind als erfindungswesentlich einzustufen und beliebig miteinander kombinierbar oder in Alleinstellung einsetzbar.

## Patentansprüche

1. Vorrichtung zur Knochenbearbeitung, umfassend einen Werkzeugkopf und ein daran angeordnetes Werkzeug, wobei der Werkzeugkopf (WK) wenigstens ein relativ zu diesem verstellbares Stützelement (2) aufweist, mittels welchem der Werkzeugkopf (WK) bei einer Knochenbearbeitung auf der Oberfläche eines zu bearbeitenden Knochen (4) abstützbar ist, wobei die Lage des Werkzeuges (1) relativ zu einer zu bearbeitenden Knochenoberfläche bei einer Knochenbearbeitung durch gesteuerte oder geregelte Verstellung des wenigstens einen Stützelementes (2) mittels eines Aktors verstellbar ist in Abhängigkeit von Daten, die während der Knochenbearbeitung einer Steuer-/Regeleinheit des wenigstens einem Aktors zuführbar sind, wobei die Vorrichtung wenigstens eine Sensorik (3) aufweist, mittels der bei der Knochenbearbeitung die Daten zur Ansteuerung des wenigstens einen Aktors erfassbar sind, wobei diese Daten Informationen über Eigenschaften am Ort oder zumindest in der Umgebung des Ortes der Knochenbearbeitung repräsentieren,
**dadurch gekennzeichnet, dass** wenigstens zwei verstellbare Stützelemente (2) am Werkzeugkopf (WK) vorgesehen sind, so dass durch Verstellung der Stützelemente (2) die Höhenlage und/oder Winkellage des Werkzeugs (1) relativ zur Knochenoberfläche bei der Bearbeitung verstellbar ist.

2. Vorrichtung nach Anspruch 1, **dadurch gekennzeichnet, dass** die wenigstens zwei Stützelemente (2) um das Werkzeug (1) herum angeordnet sind.

3. Vorrichtung nach einem der vorherigen Ansprüche, **dadurch gekennzeichnet, dass** die Vorrichtung mit einem Navigationssystem zusammenwirkt, mittels dem die Position des Werkzeugkopfes (WK) relativ zu einem zu bearbeitenden Knochen ermittelbar ist und der Vorrichtung weitere Daten in Abhängigkeit einer festgestellten Position des Werkzeugkopfes (WK) aus einer Datenbank zuführbar sind, insbesondere wobei an der Vorrichtung und/oder am Knochen mehrere durch eine Kamera oder ein anderes räumliches Lokalisierungssystem erfassbare Markierungen angeordnet sind.

4. Vorrichtung nach Anspruch 3, **dadurch gekennzeichnet, dass** die weiteren Daten vor einer Knochenbearbeitung durch messtechnische, insbesondere bildgebende Erfassung des zu bearbeitenden Knochens (4), positionsabhängig erfasst und in einer Datenbank gespeichert sind.

5. Vorrichtung nach einem der vorherigen Ansprüche 1 bis 4, **dadurch gekennzeichnet, dass** die Sensorik (3) zumindest teilweise in wenigstens einem der Stützelemente (2) angeordnet ist, insbesondere im Bereich des Endes eines Stützelementes (2), welches der Oberfläche eines zu bearbeitenden Knochens (4) zugewandt ist.

6. Vorrichtung nach einem der vorherigen Ansprüche 1 bis 4, **dadurch gekennzeichnet, dass** die Sensorik zumindest teilweise in einem Werkzeug angeordnet ist oder zumindest Messdaten über das Werkzeug erfassbar sind.

7. Vorrichtung nach einem der vorherigen Ansprüche 1 bis 6, **dadurch gekennzeichnet, dass** die Sensorik (3) ein akustisches Messprinzip aufweist, insbesondere Ultraschallsender und Ultraschallempfänger umfasst.

8. Vorrichtung nach Anspruch 7, **dadurch gekennzeichnet, dass** die von der Sensorik (3) wenigstens eines der Stützelemente (2) ausgesendeten Ultraschallimpulse von derselben Sensorik (3) und/oder der Sensorik (3) eines anderen Stützelementes (2) als Echos empfangbar sind.

9. Vorrichtung nach einem der vorherigen Ansprüche 6 oder 8, **dadurch gekennzeichnet, dass** die Sensorik (3), insbesondere im Falle der im Stützelement (2) angeordneten Sensorik (3), einen Applikator zur Applikation eines Koppelfluids aufweist.

10. Vorrichtung nach einem der vorherigen Ansprüche, **dadurch gekennzeichnet, dass** die wenigstens zwei Stützelemente (2) ausgebildet sind als Stempel, die mittels eines Aktors aus dem Werkzeugkopf (WK) aus- oder in diesen einfahrbar sind.

## Claims

1. Apparatus for working a bone, comprising a tool head and a tool carried on it, wherein the tool head (WK) has at least one support member (2) adjustable relative to it, by means of which the tool head (WK) can be supported when working a bone on the surface of a bone (4) being worked, in which the position of the tool (1) relative to a bone surface being worked when working a bone is adjustable by means of an actuator through a controlled or regulated adjustment of the at least one support member (2) depending on data which can be supplied while the bone is being worked to a control/regulation unit of the at least an actuator, in which the apparatus has at least one sensor (3), by means of which, while working a bone, the data can be captured for controlling the at least one actuator, in which this data represents information relating to properties at the location or at least around the location where the bone is being worked,
**characterized in that** at least two adjustable support members (2) are provided on the tool head (WK), such that by adjusting the support members (2) the height and/or angle position of the tool (1) can be adjusted relative to the bone surface of a bone being worked.

2. Apparatus for working a bone, **characterized in that** the at least two support members (2) are being located around the tool (1).

3. Apparatus according to one of the preceding claims, **characterized in that** the apparatus cooperates with a navigation system, by means of which the position of the tool head (WK) relative to a bone being worked can be determined, and that additional data can be supplied to the apparatus from a database depending on an observed position of the tool head (WK), in particular in which several markers capturable by a camera or other spatial location system are located on the apparatus and/or the bone.

4. Apparatus according to claim 3, **characterized in that** the additional data are captured depending on the position prior to working the bone by metrological capture, in particular imaging of the bone (4) being worked and stored in a database.

5. Apparatus according to one of the preceding claims 1 to 4, **characterized in that** the sensor (3) is at least partially located in at least one of the support members (2), in particular in the region of an end of a support member (2) facing the surface of a bone (4) that is being worked.

6. Apparatus according to one of the preceding claims 1 to 4, **characterized in that** the sensor is at least partially located in a tool or at least data can be captured by the tool.

7. Apparatus according to one of the preceding claims 1 to 6, **characterized in that** the sensor (3) has an acoustic measurement principle, in particular comprises an ultrasound transmitter and an ultrasound receiver.

8. Apparatus according to claim 7, **characterized in that** the ultrasound impulses transmitted by the sensor (3) of at least one of the support members (2) are received by the same sensor (3) and/or the sensor (3) of a different support member (2) as echoes.

9. Apparatus according to one of the preceding claims 6 or 8, **characterized in that** the sensor (3), in particular in the case of the sensor (3) located in the support member (2), has an applicator for applying a coupling fluid.

10. Apparatus according to one of the preceding claims, **characterized in that** the at least two support members (2) are formed in the form of slides which are moved out and into the tool head (WK) by an actuator.

## Revendications

1. Dispositif pour le travail d'un os, comprenant une tête d'outil et un outil agencé contre celle-ci, dans lequel la tête d'outil (WK) présente au moins un élément de support (2) réglable par rapport à celle-ci, au moyen duquel la tête d'outil (WK) peut être soutenue lors du travail d'un os sur la surface d'un os (4) à travailler, dans lequel la position de l'outil (1) par rapport à une surface d'os à travailler lors du travail d'un os est réglable au moyen d'un actionneur par un réglage commandé ou régulé du au moins un élément de support (2) en fonction de données qui peuvent être fournies lors du travail de l'os à une unité de commande/régulation du au moins un actionneur, dans lequel le dispositif présente au moins un dispositif de capteur (3), au moyen duquel lors du travail d'un os les données peuvent être capturées pour un pilotage du au moins un actionneur, dans lequel ces données représentent des informations relatives à des propriétés à l'emplacement ou au moins dans l'environnement de l'emplacement du travail d'un os,
**caractérisé en ce que** sont prévus au moins deux éléments de support (2) réglables sur la tête d'outil (WK), de sorte que par un réglage des éléments de support (2) les position de hauteur et/ou position d'angle de l'outil (1) soient réglables par rapport à la surface d'un os lors du travail d'un os.

2. Dispositif selon la revendication 1, **caractérisé en ce que** les au moins deux éléments de support (2) sont agencés autour de l'outil (1).

3. Dispositif selon l'une des revendications précédentes, **caractérisé en ce que** le dispositif coopère avec un système de navigation, au moyen duquel la position de la tête d'outil (WK) par rapport à un os à travailler peut être déterminée, et que d'autres données peuvent être fournies au dispositif depuis une banque de données en fonction d'une position constatée de la tête d'outil (WK), en particulier dans lequel plusieurs repères pouvant être capturés par une caméra ou un autre système de localisation dans l'espace sont agencés contre le dispositif et/ou contre l'os.

4. Dispositif selon la revendication 3, **caractérisé en ce que** les autres données sont capturées en fonction de la position avant le travail d'un os par une capture métrologique, en particulier d'imagerie de l'os (4) à travailler et mémorisées dans une banque de données.

5. Dispositif selon l'une des revendications précédentes 1 à 4, **caractérisé en ce que** le dispositif de capteur (3) est agencé au moins partiellement dans au moins un des éléments de sport (2), en particulier dans la région de l'extrémité d'un élément de support (2), lequel est tourné vers la surface d'un os (4) à travailler.

6. Dispositif selon l'une des revendications précédentes 1 à 4, **caractérisé en ce que** le dispositif de capteur est agencé au moins partiellement dans un outil ou au moins des données peuvent être capturées par l'outil.

7. Dispositif selon l'une des revendications précédentes 1 à 6, **caractérisé en ce que** le dispositif de capteur (3) présente un principe de mesure acoustique, en particulier comprend émetteur d'ultrasons et récepteur d'ultrasons.

8. Dispositif selon la revendication 7, **caractérisé en ce que** les impulsions ultrasonores émises par le dispositif de capteur (3) d'au moins un des éléments de support (2) peuvent être reçues sous forme d'échos par le même dispositif de capteur (3) et/ou le dispositif de capteur (3) d'un autre élément de support (2).

9. Dispositif selon l'une des revendications précédentes 6 ou 8, **caractérisé en ce que** le dispositif de capteur (3), en particulier dans le cas du dispositif de capteur (3) agencé dans l'élément de support (2), présente un applicateur pour l'application d'un fluide de couplage.

10. Dispositif selon l'une des revendications précédentes, **caractérisé en ce que** les au moins deux éléments de support (2) sont formés sous forme de pistons qui peuvent être conduits au moyen d'un actionneur depuis la tête d'outil (WK) hors de ou dans celle-ci.
